**Europäisches Patentamt**

**European Patent Office**

**Office européen des brevets**

(19)

(11) Publication number : **0 348 781 B1**

# EUROPEAN PATENT SPECIFICATION

(12)

(45) Date of publication of patent specification :
**30.09.92 Bulletin 92/40**

(51) Int. Cl.[5] : **A61K 35/78,** A61K 31/35,
C07D 311/32

(21) Application number : **89111095.9**

(22) Date of filing : **19.06.89**

(54) **Procyanidol oligomeric fractions, the processes for the preparation thereof and pharmaceutical compositions containing them.**

(30) Priority : **28.06.88 IT 2113488**

(43) Date of publication of application :
**03.01.90 Bulletin 90/01**

(45) Publication of the grant of the patent :
**30.09.92 Bulletin 92/40**

(84) Designated Contracting States :
**AT BE CH DE ES FR GB GR IT LI LU NL SE**

(56) References cited :
**FR-A- 2 092 743**
**FR-A- 2 268 518**

(73) Proprietor : **TECNOFARMACI S.p.A.**
**Piazza Indipendenza, 24**
**I-00040 Pomezia (Rome) (IT)**
Proprietor : **INDENA S.p.A.**
**Via Ripamonti, 99**
**I-20141 Milano (IT)**

(72) Inventor : **Frangi, Enrico**
**Via Ripamonti, 99**
**I-20141 Milano (IT)**
Inventor : **Bertani, Marco**
**Via Ripamonti, 99**
**I-20141 Milano (IT)**
Inventor : **Mustich, Giuseppe**
**Via Ripamonti, 99**
**I-20141 Milano (IT)**
Inventor : **Tuccini, Gianfranco**
**Via Ripamonti, 99**
**I-20141 Milano (IT)**

(74) Representative : **Bianchetti, Giuseppe**
**Studio Consulenza Brevettuale Via Rossini, 8**
**I-20122 Milan (IT)**

## Description

The present invention relates to novel vegetal extracts containing procyanidol oligomeric fractions, to the processes for the preparation thereof and to pharmaceutical compositions containing them.

The extracts from Vitis vinifera grapeseeds, containing said oligomers, have already been used in therapy, particularly as vasoprotectors in the treatment of disorders of the retinal or choroidal circulation and of venolymphatic insufficiencies; in fact, they increase the resistance of vessels and capillaries and decrease the permeability thereof.

Procyanidol oligomers are polyphenols deriving from flavanols, resulting from the oxidative condensations of various flavanolic units, which can be the same or different, particularly from the condensation of (+) catechin, epicatechin and gallocatechin. They are present in different vegetals, such as vine, appletree, maritime pine, hawthorn, cinchona and eucalyptus.

The extracts prepared according to the processes of the prior art, particularly those disclosed in FR-A-2.092.743 and FR-A-2.372.823 concerning Vitis vinifera, or those disclosed in FR 1.427.100 and in USA Pat. 4.698.360 concerning maritime pine, proved to contain, in addition to the oligomers of polymerization degree 2 to 5, condensation derivatives of higher molecular weights, in spite of a treatment with a sodium chloride saturated aqueous solution, and, above all, monomers in the not neglectable proportions of 20 to 30% by weight. These latter are considered undesired substances.

The extracts according to the present invention, resulting from a suitably selected treatment of the extracts of the prior art, contain less than 1,5% by weight of monomers and show a higher pharmacological activity than that of the starting extracts, as they are enriched in the procyanidol oligomeric fraction.

Although a number of processes for the extraction of flavanolic oligomers or similar substances present in vegetal material have already been described (see, for instance, FR Patents 968.589, 1.036.922 and 1.427.100 and GB Patent 1.541.469), it is not obvious to obtain on industrial scale and in economically acceptable yields extracts almost completely free from monomers, with no remarkable losses or alterations in the dimers and the other oligomers, whose physico-chemical characteristics are clearly similar: the process of the present invention overcomes this problem for the first time.

According to the process of the present invention, the monomers are separated either by ultrafiltration of an aqueous solution of the extracts obtained according to conventional processes, on membranes of cut-off 600 to 3.000, or by selective extraction of the above extracts with an ether, such as methyl-tert-butyl ether or with a water immiscible mixture of an aliphatic ester, such as ethyl acetate or amyl acetate, or an ether, such as methyl-tert-butyl ether, with an aromatic hydrocarbon, such as benzene or toluene.

Generally speaking, the processes of the invention are characterized by the following steps:

A) Extraction of the vegetal material with mixtures of water with acetone, methanol or other alcohols, till exhaustion in catechin derivatives; 80% aqueous acetone is used as the solvent mixture of choice.

B) Concentration of the solvent by evaporation under vacuum to a weight approximately 50% of the starting weight of the vegetal material.

C) Filtration of the concentrate from step B), optionally after standing in refrigerator for 24 hours, to remove any oleaginous and tannic polymeric residues.

D) Ultrafiltration of the concentrate from step C), under nitrogen atmosphere, through tubular or capillary membranes of cut-off 100.000 to 1.000.

E) Ultrafiltration of the permeate from step D), from which the high molecular weight substances have been removed, through coiled-spiral membranes of cut-off 3.000 to 600.

F) Optional further fractionation of permeate from step E) by ultrafiltration, limiting the cut-off of the membranes, according to hereinafter reported procedures, or subjecting the permeate to fractionation with selective organic solvents.

The permeate from step D) above, before the final purifications (however they are carried out), still contains monomeric substances: in the case of Vitis vinifera (+)-catechin and (-)-epicatechin and, in lower amounts, undesired polymeric substances. In order to obtain a good demonomerization degree, the more convenient process comprises a further ultrafiltration on coiled-spiral membranes of cut-off 600 to 3.000 and counter-extraction of the concentrate with ethyl acetate; the final demonomerized and detannized product is obtained by evaporating the organic phase to reduced volume and insolubilizating the concentrate in chloroform or other chlorinated solvents, such as methylene chloride or dichloroethane. Alternatively, the aqueous solution from step C) can directly be extracted with ethyl acetate, and the residue from the organic phase, after substitution with water, is subjected to the membrane processes of steps D) and/or E); the aqueous solution from step C), adjusted to a concentration from 10 to 50% can be extracted with solvent mixtures consisting of ethyl acetate/toluene or other aromatic hydrocarbons, in ratios of 5 to 9 parts of the first solvent and 5 to 1 parts of the second one; extraction is continued with said mixtures until exhaustion in monomeric substances. After an appropriate ratio between the two solvents has been reached, based on the partition coefficients, the organic phase is counter-extracted with water to recover the amount of dimeric compounds which could accidentally have been extracted by co-solubilization; the combined aqueous phases are extracted with pure ethyl acetate, the organic phase is evaporated under vacuum to reduced final volume and the concentrate is precipitated in a chlorinated solvent.

Alternatively, the mixtures of ethyl acetate with hydrocarbons can advantageously be replaced by other esters, such as isopropyl acetate, amyl acetate or ethyl formate, or aliphatic ethers, methyl-tert-butyl ether being preferred. Esters, as well as ethers, selectively remove undesired monomeric fractions, besides any lipophilic materials which are usually present in vegetal extracts; the subsequent extraction with ethyl acetate is selective for the oligomeric substances against the polymeric ones, whereby, according to one of the above processes, a product almost free from monomers, which can be allergenic, as well as from polymers having a tanning action, which are irritant for mucosae, can be obtained.

By taking advantage of the selectivity of the above cited solvents and mixtures thereof, extracts having the same chemical characteristics can be prepared carrying out the fractionation by means of a solid matrix, which selectively absorbs the phenol derivatives, which can be re-eluted from said matrices by homogeneous classes.

A polystyrene resin, such as the absorption Amberlites[R] or preferably a Duolite[R] S-761 can be used as the solid matrix. According to this procedure, solution from step C) is passed through a column of the selected resin, using about 0,5 liter of support for each 10 g of dry residue of the solution; when the absorption of the starting solution is over the resin is thoroughly washed with water to completely remove salts, sugars, organic acids and heavy polymeric fractions, which are excluded due to the steric hindrance thereof; washing of the resin is continued with a mixture of ethyl acetate and aliphatic or aromatic hydrocarbons, preferably in a 7:3 ratio, till the monomeric substances are completely removed. The extract of the invention is obtained by subsequent elution with aqueous methanol or aqueous acetone, the resulting eluate being concentrated to water. The aqueous concentrate is extracted according to the above reported procedures with ethyl acetate and the residue, after concentration, is precipitated by means of chlorinated solvents.

The extract obtained according to the above disclosed processes shows, in comparison with the prior art ones, a perfect tolerability and a very good activity on the pathologies connected to the cardiovascular system and to superficial and deep blood circle. This extract is suited for incorporation in all the usual liquid and solid pharmaceutical formulations.

The following non-limiting examples illustrate the invention in more detail.

## EXAMPLE 1

Preparation of the demonomerized extract starting from Vitis vinifera dry grapeseeds.

100 kg of Vitis vinifera grapeseeds are soaked in 150 l of acetone containing 20% water, at room temperature. The extraction solvent is filtered and the extraction is repeated for 4 times more or till exhaustion in phenol derivatives, checking with $FeCl_3$ an aliquot from the last extract and verifying dry residue is less than 0,05%. Hydroacetone solutions are combined and acetone is evaporated off by distillation under vacuum until the organic solvent is completely removed; during the final concentration an abundant precipitate forms which, after standing in refrigerator at 4°C for 24 hours, is centrifuged; this solid comprises lipophilic products and highly polymerized tannic material. The aqueous solution (43 l) is adjusted to pH 6 with 40% NaOH, under nitrogen, and ultrafiltered on a tubular membrane of cut-off 100.000 Enichem® (UF-100F), at a temperature below 20°C, and dialyzed with 57 l of water. The permeate, from which polymers have been removed, is acidified with 25% $H_2SO_4$ to pH 3,5 and concentrated by reverse osmosis with coiled-spiral membrane Desal® B241-111 till a 30% dry residue. The aqueous concentrate is extracted continuously with an ethyl acetate:toluene 8:2 (v/v) mixture by means of a liquid/liquid centrifuge separator till exhaustion in monomeric substances; the exhausted solution is extracted continuously with ethyl acetate until exhaustion in oligomers. The organic phase is concentrated under vacuum to 1,5 liter and precipitated with 4,5 l of methylene chloride. The product is centrifuged which, after drying for 24 hours at 50°C under vacuum, weighed 0,42 kg. This purified extract contains the monomeric substances in a <1% amount.

## EXAMPLE 2

100 kg of Vitis vinifera grapeseeds are extracted according to the procedure of Example 1. The permeate, after ultrafiltration on a tubular membrane with cut-off 100.000( Enichem® UF-100F) is acidified to pH 3,5 and ultrafiltered on a coiled-spiral membrane Desal® U20-G20 and the solution is dialyzed with 20 volumes of water with respect to the starting solution volume. The concentrate (from which the monomeric substances have been removed) is further concentrated to a 30% dry residue. The aqueous concentrate is extracted with ethyl acetate till exhaustion in the oligomers; the organic phase, after concentration to 1,5 l, is poured into 4,5 l of methylene chloride. The precipitated oligomers are filtered and dried at 50°C for 24 hours, to obtain 0,45 kg of the same final product as in Example 1.

## EXAMPLE 3

100 kg of Vitis vinifera grapeseeds are extracted according to the procedure of Example 1. The aqueous solution, after centrifugation of the undesired polymeric substances, is continuously extracted with ethyl acetate in liquid/liquid centrifuge separator, until exhaustion in the phenol substances which can be extracted. The organic phase is concentrated, adding demineralized water to remove the solvent, reaching a final volume of 150 l.

The aqueous solution at pH 3,5 can be subjected either to the treatment reported in Example 1, using tubular membranes of cut-off 100.000 and reverse osmosis, or to a treatment according to the procedure of Example 2, with a membrane of cut-off 100.000 and a coiled-spiral membrane Desal® U20-G20. The concentrated aqueous solutions are treated according to the procedures of Examples 1 and 2.

## EXAMPLE 4

100 kg of Vitis vinifera grapeseeds are extracted according to the procedure of Example 1. The aqueous solution, after filtrating off the undesired polymeric fraction, is continuously extracted with ethyl acetate till exhaustion in the polyphenols which can be extracted with said solvent; the organic phase is concentrated to a pasty consistence and the residue is dissolved in 20 l of water, the residual solvent being removed under vacuum. The aqueous solution is absorbed on a column containing 30 l of the polysterene resin Duolite® S-761, continuing the elution until obtaining nearly discolored water. Then elution is continued with an ethyl acetate/toluene 7:3 mixture till complete remotion of the monomeric substances, checking the reaction by thin layer chromatography, using a butyl acetate/acetic acid/formic acid/water 8:1:1:1 mixture as the eluent and chlorosulfonic acid in acetic acid as the developer. The oligomeric fraction is obtained by continuing elution with almost pure ethyl acetate. The organic solution containing mainly dimeric, trimeric and tetrameric substances is concentrated to 1 liter and poured into 3 l of methylene chloride. After filtration and drying, 210 g of a product containing monomers in less than 0,1% are obtained.

Alternatively, the column is washed with methanol instead of pure ethyl acetate; the alcoholic phase is concentrated to small volume and the residue is diluted with 30 l of water; the aqueous phase is counter-extracted with ethyl acetate, following the procedure of Examples 1 and 2 to obtain the product. In this instance, 405 g of the product are obtained, which is nearly the same as the above described ones.

## EXAMPLE 5

100 kg of Vitis vinifera grapeseeds are extracted according to the procedure of Example 1. After centrifugation of the polymeric substances, the aqueous solution having an about 8% dry residue is diluted to reach an about 5% dry residue and chromatographed on a column containing 50 l of Duolite® S-761, thoroughly washing with about 500 l of water, so as to completely remove salts and sugars which are abundantly present in the extract. Then, purification of the oligomers is carried out as in Example 4, washing the column first with ethyl acetate/toluene, then with methanol; after precipitation from methylene chloride, 423 g of an oligomeric fraction which is similar to that from the other Examples are obtained.

## EXAMPLE 6

100 kg of Vitis vinifera grapeseeds are extracted according to the procedure of Example 1; the aqueous solution after filtrating off the polymeric material is extracted with 10 x 50 l portions of an ethyl acetate/toluene 8:2 mixture; the organic phase, containing fatty materials and the monomers, is counter-washed with 20 l of water; the combined aqueous phases are extracted 10 x 50 l portions of ethyl acetate saturated with water; the organic phase is concentrated to a pasty consistence and the residue is taken up into 7 l of water, the residual solvent being evaporated under vacuum. The slightly turbid aqueous solution is treated with 750 g of NaCl; a precipitate forms which, after standing of the solution in refrigerator for 24 hours, is filtered. The filtrate is extracted with 10 x 7 l portions of ethyl acetate; the organic solution is concentrated to 1 liter and the concentrate is poured into 3 l of methylene chloride. After filtration and drying according to the procedures described in the above Examples, 402 g of a final product are obtained, which is the same as the already described ones.

## EXAMPLE 7

100 kg of Vitis vinifera grapeseeds are extracted according to the procedure of Example 2. The permeate from the coiled-spiral membrane Desal® U20-G20 of Example 2 and/or the aqueous solution obtained from the ethyl acetate/toluene 8:2 mixture according to the procedure of Example 1, after concentration to a pasty residue and substitution of the solvent with water, are ultrafiltered on a coiled-spiral membrane U20-G5. The concentrated aqueous solution, after dialysis, is nearly free from monomers and contains the oligomers which are extracted with ethyl acetate. The organic solution is concentrated to 0,1 liter and precipitated with 0,5 l of methylene chloride.

3,81 g of a product containing 38% dimeric substances and about 1% monomeric catechins are obtained.

## Claims

**Claims for the following Contracting States : AT, BE, CH, DE, FR, GB, IT, LI, NU, NL, SE**

1. Vegetal extracts containing procyanidol oligomers, and less than 1,5% by weight in flavanolic monomers.

2. Vegetal extracts as claimed in claim 1, containing less than 1% by weight in (+)-catechin.

3. Vegetal extracts as claimed in claim 1 and 2, extracted from Vitis vinifera.

4. A process for the preparation of the vegetal extracts as claimed in claims 1 to 3, in which process vegetal extracts obtained by per se known methods, are freed from the high molecular weight substances by means of precipitation in water and subsequent ultrafiltration on membranes of cut-off 1.000 to 100.000, then are freed from the monomers by ultrafiltration on membranes of cut-off less than 3.000.

5. A process as claimed in claim 4, in which membranes of cut-off 3.000 to 600 are employed to remove the monomers.

6. A process as claimed in claims 4 and 5, in which tubular, capillary, coiled-spiral or plane membranes are used for the ultrafiltration.

7. A process as claimed in claims 4 to 6, in which the permeate from ultrafiltration on membranes of cut-off 3.000 to 600 is subjected to a further ultrafiltration on membranes with a more limited cut-off.

8. A process as claimed in claims 4 to 7, in which the permeate from ultrafiltration is concentrated, then subjected to exhausting extraction with ethyl acetate, the oligomers being finally recovered from the organic phase by concentration of said phase and precipitation with chlorinated hydrocarbons.

9. A process for the preparation of the vegetal extracts as claimed in claims 1 to 3, in which process the vegetal extracts, obtained by per se known methods, are freed from the high molecular weight substances by precipitation with water and subsequent ultrafiltration on membranes of cut-off 100.000 to 1.000, then freed from the flavanole monomers by selective extraction in a water-immiscible mixture of an aliphatic ester and an aromatic hydrocarbon or an appropriate ether.

10. A process as claimed in claim 9, in which the extraction solvent consists of 5 to 9 parts by volume of ethyl acetate and 5 to 1 parts by volume of toluene.

11. A process as claimed in claims 9 and 10, in which the extraction is carried out by eluting with the above solvent the flavanol extract deposited on a chromatographic column.

12. Pharmaceutical compositions containing as the active ingredient the vegetal extracts as claimed in claims 1 to 3.

**Claims for the following Contracting States : ES, GR**

1. A process for the preparation of vegetal extracts containing procyanidol oligomers, and less than 1,5% by weight in flavanolic monomers, in which process said vegetal extracts, obtained by per se known methods, are freed from the high molecular weight substances by means of precipitation in water and subsequent ultrafiltration on membranes of cut-off 1.000 to 100.000, then are freed from the monomers by ultrafiltration on membranes of cut-off less than 3.000.

2. A process as claimed in claim 1, in which membranes of cut-off 3.000 to 600 are employed to remove the monomers.

3. A process as claimed in claims 1 and 2, in which tubular, capillary, coiled-spiral or plane membranes are used for the ultrafiltration.

4. A process as claimed in claims 1 to 3, in which the permeate from ultrafiltration on membranes of cut-off 3.000 to 600 is subjected to a further ultrafiltration on membranes with a more limited cut-off.

5. A process as claimed in claims 1 to 4, in which the permeate from ultrafiltration is concentrated, then subjected to exhausting extraction with ethyl acetate, the procyanidol oligomers being finally recovered from the organic phase by concentration of said phase and precipitation with chlorinated hydrocarbons.

6. A process for the preparation of vegetal extracts containing procyanidol oligomers and less than 1,5% by weight in flavonolic monomers in which process the vegetal extracts, obtained by per se known methods, are freed from the high molecular weight substances by precipitation with water and subsequent ultrafiltration on membranes of cut-off 100.000 to 1.000, then freed from the flavanol monomers by selective extraction in a water-immiscible mixture of an aliphatic ester and an aromatic hydrocarbon or an appropriate ether.

7. A process as claimed in claim 6, in which the extraction solvent consists of 5 to 9 parts by volume of ethyl acetate and 5 to 1 parts by volume of toluene.

8. A process as claimed in claims 6 and 7, in which the extraction is carried out by eluting with the above solvent the flavanol extract. deposited on a chromatographic column.

**Patentansprüche**

**Patentansprüche für folgende Vertragsstaaten : AT, BE, CH, DE, FR, GB, IT, LI, LU, NL, SE**

1. Pflanzliche Extrakte, enthaltend Procyanidol-Oligomere und weniger als 1,5 Gew.-% an Favanol-Monomeren.

2. Pflanzliche Extrakte nach Anspruch 1, enthaltend weniger als 1 Gew.-% (+)-Catechin.

3. Pflanzliche Extrakte nach Anspruch 1 und 2, extrahiert aus Vitis vinifera.

4. Verfahren zur Herstellung der pflanzlichen Extrakte nach den Ansprüchen 1 bis 3, wobei bei diesem Verfahren nach an sich bekannten Methoden erhaltene pflanzliche Extrakte von den Substanzen mit hohem Molekulargewicht befreit werden durch Ausfällen in Wasser und anschließende Ultrafiltration an Membranen mit einer Ausschlußgrenze von 1000 bis 100000, und anschließend von den Monomeren durch Ultrafiltration an Membranen mit einer Ausschlußgrenze von weniger als 3000 befreit werden.

5. Verfahren nach Anspruch 4, bei dem Membranen mit einer Ausschlußgrenze von 3000 bis 600 zur Entfernung der Monomeren verwendet werden.

6. Verfahren nach den Ansprüchen 4 und 5, bei dem rohrförmige, kapillarartige, gewickelt-spiralförmige oder ebene Membranen für die Ultrafiltration verwendet werden.

7. Verfahren nach den Ansprüchen 4 bis 6, bei dem das Permeat der Ultrafiltration an Membranen mit einer Ausschlußgrenze von 3000 bis 600 einer weiteren Ultrafiltration an Membranen mit einer begrenzteren Ausschlußgrenze unterzogen werden.

8. Verfahren nach den Ansprüchen 4 bis 7, bei dem das Permeat der Ultrafiltration konzentriert und anschließend einer erschöpfenden Extraktion mit Ethylacetat unterzogen wird, wobei die Oligomeren schließlich aus der organischen Phase durch Konzentration dieser Phase und Ausfällen mit chlorierten Kohlenwasserstoffen gewonnen werden.

9. Verfahren zur Herstellung der pflanzlichen Extrakte nach den Ansprüchen 1 bis 3, wobei bei dem Verfahren die nach an sich bekannten Methoden erhaltenen Extrakte von den Substanzen mit hohem Molekulargewicht befreit werden durch Ausfällen mit Wasser und anschließende Ultrafiltration an Membranen mit einer Ausschlußgrenze von 100000 bis 1000, und anschließend von den Flavanol-Monomeren durch selektive Extraktion in einem mit Wasser nicht mischbaren Gemisch eines aliphatischen Esters und eines aromatischen Kohlenwasserstoffs oder eines geeigneten Ethers befreit werden.

10. Verfahren nach Anspruch 9, bei dem das Extraktionslösungsmittel aus 5 bis 9 Volumenteilen Ethylacetat und 5 bis 1 Volumenteilen Toluol besteht.

11. Verfahren nach den Ansprüchen 9 und 10, bei dem die Extraktion durch Eluieren des an einer Chromatographiesäule abgelagerten Flavanolextrakts mit dem vorstehenden Lösungsmittel durchgeführt wird.

12. Pharmazeutische Zusammensetzungen, enthaltend als aktiven Bestandteil die pflanzlichen Extrakte gemäß den Ansprüchen 1 bis 3.

**Patentansprüche für folgende Vertragsstaaten : ES, GR**

1. Verfahren zur Herstellung pflanzlicher Extrakte, die Procyanidol-Oligomere und weniger als 1,5 Gew.-% Favanol-Monomere enthalten, wobei bei diesem Verfahren die nach an sich bekannten Methoden erhaltenen pflanzlichen Extrakte von den Substanzen mit hohem Molekulargewicht befreit werden durch Ausfällen in Wasser und anschließende Ultrafiltration an Membranen mit einer Ausschlußgrenze von 1000 bis 100000, und anschließend von den Monomeren durch Ultrafiltration an Membranen mit einer Ausschlußgrenze von weniger als 3000 befreit werden.

**2.** Verfahren nach Anspruch 1, bei dem Membranen mit einer Ausschlußgrenze von 3000 bis 600 zur Entfernung der Monomeren verwendet werden.

**3.** Verfahren nach den Ansprüchen 1 und 2, bei dem rohrförmige, kapillarförmige, gewickelt-spiralförmige oder ebene Membranen zur Ultrafiltration verwendet werden.

**4.** Verfahren nach den Ansprüchen 1 bis 3, bei dem das Permeat der Ultrafiltration an Membranen mit einer Ausschlußgrenze von 3000 bis 600 einer weiteren Ultrafiltration an Membranen mit einer begrenzteren Ausschlußgrenze unterzogen werden.

**5.** Verfahren nach einem der Ansprüche 1 bis 4, bei dem das Permeat der Ultrafiltration konzentriert und anschließend einer erschöpfenden Extraktion mit Ethylacetat unterzogen wird, wobei die Procyanidol-Oligomeren schließlich aus der organischen Phase durch Konzentrieren dieser Phase und Ausfällen mit chlorierten Kohlenwasserstoffen gewonnen werden.

**6.** Verfahren zur Herstellung pflanzlicher Extrakte, die Procyanidol-Oligomere und weniger als 1,5 Gew.-% Flavanol-Monomere enthalten, wobei bei dem Verfahren die nach an sich bekannten Methoden erhaltenen pflanzlichen Extrakte von den Substanzen mit hohem Molekulargewicht befreit werden durch Ausfällen mit Wasser und anschließende Ultrafiltration an Membranen mit einer Ausschlußgrenze von 100000 bis 1000, und anschließend von den Flavanol-Monomeren durch selektive Extraktion in einem mit Wasser nicht mischbaren Gemisch eines aliphatischen Esters und eines aromatischen Kohlenwasserstoffs oder eines geeigneten Ethers befreit werden.

**7.** Verfahren nach Anspruch 6, bei dem das Extraktionslösungsmittel aus 5 bis 9 Volumenteilen Ethylacetat und 5 bis 1 Volumenteilen Toluol besteht.

**8.** Verfahren nach den Ansprüchen 6 und 7, bei dem die Extraktion durch Eluieren des auf einer Chromatographiesäule abgelagerten Flavanolextrakts mit dem vorstehenden Lösungsmittel durchgeführt wird.

**Revendications**

**Revendications pour les Etats contractants suivants : AT, BE, CH, DE, FR, GB, IT, LI, LU, NL, SE**

1 - Extraits végétaux contenant des oligomères de procyanidol et moins de 1,5% en poids de monomères flavanoliques.

2 - Extraits végétaux selon la revendication 1, contenant moins de 1% en poids de (+)-catéchine.

3 - Extraits végétaux selon l'une des revendications 1 et 2, extraits de Vitis vinifera.

4 - Procédé de préparation des extraits végétaux tels que revendiqués dans les revendications 1 à 3, procédé dans lequel des extraits végétaux, obtenus par des méthodes connues en soi, sont libérés des substances de masse moléculaire élevée par une précipitation dans l'eau et ultrafiltration ultérieure sur des membranes présentant un seuil de coupure de 1000 à 100 000, puis sont libérés des monomères par ultrafiltration sur des membranes présentant un seuil de coupure inférieur à 3000.

5 - Procédé selon la revendication 4, dans lequel des membranes présentant un seuil de coupure de 3000 à 600 sont employées pour éliminer les monomères.

6 - Procédé selon les revendications 4 et 5, dans lequel des membranes tubulaires, capillaires, enroulées en spirale ou planes sont utilisées pour l'ultrafiltration.

7 - Procédé selon l'une des revendications 4 à 6, dans lequel le perméat provenant de l'ultrafiltration sur des membranes présentant un seuil de coupure de 3000 à 600 est soumis à nouveau à une ultrafiltration sur des membranes présentant un seuil de coupure plus limité.

8 - Procédé selon l'une des revendications 4 à 7, dans lequel le perméat provenant de l'ultrafiltration est concentré, puis soumis à une extraction d'épuisement à l'acétate d'éthyle, les oligomères étant finalement récupérés à partir de la phase organique par concentration de ladite phase et précipitation par des hydrocarbures chlorés.

9 - Procédé de préparation des extraits végétaux tels que revendiqués dans les revendications 1 à 3, procédé dans lequel les extraits végétaux, obtenus par des méthodes connues en soi, sont libérés des substances de masse moléculaire élevée par précipitation par l'eau et ultrafiltration ultérieure sur des membranes présentant un seuil de coupure de 100 000 à 1000, puis libérés des monomères flavanoliques par extraction sélective dans un mélange non miscible à l'eau d'un ester aliphatique et d'un hydrocarbure aromatique ou d'un éther approprié.

10 - Procédé selon la revendication 9, dans lequel le solvant d'extraction consiste en 5 à 9 parties en volume d'acétate d'éthyle et 5 à 1 parties en volume de toluène.

11 - Procédé selon l'une des revendications 9 et 10, dans lequel l'extraction est effectuée par élution par le solvant ci-dessus de l'extrait de flavanol déposé sur une colonne chromatographique.

12 - Compositions pharmaceutiques contenant, comme ingrédient actif, les extraits végétaux tels que revendiqués dans les revendications 1 à 3.

**Revendications pour les Etats contractants suivants : ES, GR**

1 - Procédé de préparation d'extraits végétaux contenant des oligomères de procyanidol et moins de 1,5% en poids de monomères flavanoliques, procédé dans lequel lesdits extraits végétaux, obtenus par des méthodes connues en soi, sont libérés des substances de masse moléculaire élevée par précipitation dans l'eau et d'une ultrafiltration ultérieure sur des membranes présentant un seuil de coupure de 1000 à 100 000, puis sont libérés des monomères par ultrafiltration sur des membranes présentant un seuil de coupure inférieur à 3000.

2 - Procédé selon la revendication 1, dans lequel des membranes présentant un seuil de coupure de 3000 à 600 sont employées pour éliminer les monomères.

3 - Procédé selon les revendications 1 et 2, dans lequel des membranes tubulaires, capillaires, enroulées en spirale ou planes sont utilisées pour l'ultrafiltration.

4 - Procédé selon les revendications 1 à 3, dans lequel le perméat provenant de l'ultrafiltration sur des membranes présentant un seuil de coupure de 3000 à 600 est soumis à nouveau à une ultrafiltration sur des membranes présentant un seuil de coupure plus limité.

5 - Procédé selon les revendications 1 à 4, dans lequel le perméat provenant de l'ultrafiltration est concentré, puis soumis à une extraction d'épuisement à l'acétate d'éthyle, les oligomères de procyanidol étant finalement récupérés à partir de la phase organique par concentration de ladite phase et précipitation par des hydrocarbures chlorés.

6 - Procédé de préparation d'extraits végétaux contenant des oligomères de procyanidol et moins de 1,5% en poids de monomères flavanoliques, procédé dans lequel les extraits végétaux, obtenus par des méthodes connues en soi, sont libérés des substances de masse moléculaire élevée par précipitation par l'eau et ultrafiltration ultérieure sur des membranes présentant un seuil de coupure de 1000 000 à 1000, puis libérés des monomères flavanoliques par extraction sélective dans un mélange non miscible à l'eau d'un ester aliphatique et d'un hydrocarbure aromatique ou d'un éther approprié.

7 - Procédé selon la revendication 6, dans lequel le solvant d'extraction consiste en 5 à 9 parties en volume d'acétate d'éthyle et 5 à 1 parties en volume de toluène.

8 - Procédé selon l'une des revendications 6 et 7, dans lequel l'extraction est effectuée par élution par le solvant ci-dessus de l'extrait de flavanol déposé sur une colonne chromatographique.